# EUROPÄISCHE PATENTSCHRIFT

(11) **EP 3 653 288 B1**
(45) Veröffentlichungstag und Bekanntmachung des Hinweises auf die Patenterteilung: **29.06.2022**
(21) Anmeldenummer: 18206024.4
(22) Anmeldetag: 13.11.2018
(51) Int. Cl.: B01J 3/04, A61L 2/07

(54) **DAMPFSTERILISATOR UND VERFAHREN ZU DESSEN BETRIEB**
STEAM STERILISATION DEVICE AND METHOD FOR ITS OPERATION
STÉRILISATEUR À VAPEUR ET SON PROCÉDÉ DE FONCTIONNEMENT

(43) Veröffentlichungstag der Anmeldung: 20.05.2020
(73) Patentinhaber: MELAG Medizintechnik GmbH & Co. KG, 10829 Berlin (DE)
(72) Erfinder: BECKER, Tino, 10117 Berlin (DE); SCHLEU, Alexander, 14776 Brandenburg an der Havel (DE)
(74) Vertreter: Maikowski & Ninnemann Patentanwälte Partnerschaft mbB

(56) Entgegenhaltungen:
- DE-A1- 4 445 054
- DE-T2- 69 423 569
- DE-T2- 69 622 316

## Beschreibung

Die vorliegende Erfindung betrifft einen Dampfsterilisator gemäß dem Anspruch 1 sowie ein Verfahren zum Betrieb eines solchen Dampfsterilisators gemäß dem Anspruch 8.

Bei derartigen Dampfsterilisatoren, wie etwa Autoklaven der Klasse B, wird der zur Sterilisation eingesetzte Dampf während und nach der Sterilisationsvorgangs durch eine Vakuumpumpe aus einer Sterilisationskammer des Dampfsterilisators gesaugt. In dem abgesaugten Dampf ist dabei noch verhältnismäßig viel Wärmeenergie enthalten. Aus dem Stand der Technik sind verschiedene Möglichkeiten bekannt, den abgesaugten Dampf abzukühlen und teilweise auch die im Dampf enthaltene Wärme wiederzugewinnen.

Aus der DE 44 45 054 A1 ist ein Dampfsterilisator bekannt, bei dem ein Kondensator zwischen einer Sterilisationskammer und einer Vakuumpumpe angeordnet ist. Die Vakuumpumpe ist als Membranpumpe und der Kondensator ist als Luftkühler ausgestaltet, so dass die Vakuumpumpe und der Kondensator wasserfrei betrieben werden, um den Wasserverbrauch gering zu halten. Dabei wird lediglich beschrieben, wie Dampf aus einem Druckkessel mittels einer Vakuumpumpe abgesaugt werden kann. So wird der Dampf zunächst durch eine Kondensatorschlange durch einen Speisewasserbehälter geführt, sodass eine Erwärmung des Speisewassers erfolgen kann. Der derart abgekühlte Dampf wird dann durch einen Luftkühler gesaugt. Es wird in jener deutschen Patentanmeldung allerdings nicht beschrieben, wie unter Überdruck stehender Dampf aus dem Druckkessel ohne Einsatz einer Vakuumpumpe abgelassen werden kann.

Aus der EP 1 064 954 A1 ist ein Dampfsterilisator mit einem Druckkessel und einem Kondensator für aus dem Druckkessel stammenden Dampf bekannt. Dabei ist zusätzlich ein Mittel zur Entleerung von Kondensat aus dem Kondensator mittels Überdruck vorgesehen. Es wird auch die Möglichkeit beschrieben, erzeugtes Kondensat wieder als Speisewasser zu verwenden; eine entsprechende Kondensatleitung und ein Speisewasserbehälter sind offen miteinander verbunden, um einen Stoffaustausch zu ermöglichen.

Aus der DE 699 26 673 T2 ist ein Dampfsterilisator bekannt, bei dem Dampf aus einem Druckkessel zur Abkühlung durch eine sogenannte Spule durch einen Speisewassertank geleitet oder mittels eines Luftkühlers abgekühlt werden kann. Damit sieht dieser deutsche Teil eines europäischen Patents die Möglichkeit vor, ausschließlich eine Luftkühlung des anfallenden Dampfs vorzunehmen, wobei diese Luftkühlung immer dann zum Einsatz kommt, wenn eine Pumpe zum Absaugen des Dampfs verwendet wird.

Aus der WO 2013/021293 A1 ist ein Dampfsterilisator bekannt, bei dem zwei unterschiedliche Speisewasserpumpen vorgesehen sind. Eine erste Speisewasserpumpe dient dazu, Speisewasser aus einem Speisewassertank durch einen Kondensatsammeltank wieder zurück in den Speisewassertank zu pumpen. Eine zweite Pumpe ist vorgesehen, um Speisewasser aus dem Speisewassertank in einen Druckkessel des Dampfsterilisators zu pumpen. Mittels des Speisewasserkreislaufs, der durch die erste Speisewasserpumpe aufgebaut werden kann, kann eine Erwärmung des Speisewassers erfolgen, da es in diesem Kreislauf durch zwei Wärmeübertrager geleitet wird.

Aus der WO 2017/082962 A1 ist ein Verfahren zur Reduzierung der Temperatur eines aus einer Sterilisationskammer stammenden Dampfs bekannt. Die dem Dampf gemäß diesem Verfahren entzogene Wärme wird allerdings nicht wiederverwertet.

Aus der WO 2013/052287 A1 ist ein Dampfsterilisator bekannt, bei dem aus einer Sterilisationskammer stammender Dampf durch einen Wärmeübertrager geleitet wird. Dabei kann es zu einer Wärmeübertragung zwischen dem Dampf und Speisewasser, aus dem nachfolgend Dampf erzeugt werden soll, kommen. Dieser Wärmeübergang ist allerdings nur dann möglich, wenn das Speisewasser durch eine entsprechende Leitung strömt, also gerade zur Erzeugung von Dampf eingesetzt wird. Andernfalls kann über den Wärmeübertrager keine Wärme aus dem Dampf abgegeben werden.

Aus der DE 10 2007 046 854 B3 ist ein Verfahren zur Reduzierung des Energieverbrauchs einer Dampfbehandlungsanlage im Chargenbetrieb bekannt. Dabei werden mehrere parallel zueinander geschaltete Energiespeicher eingesetzt. Mittels eines direkten Stoffkontakts zwischen abzukühlendem Dampf und den in den Energiespeichern enthaltenen Substanzen kommt es dann zu einer Wärmeübertragung aus dem Dampf auf diese Substanzen.

Aus der US 4,708,849 A ist ein Dampfsterilisator bekannt, bei dem aus einem Druckkessel stammender Dampf entweder durch einen Wärmeübertrager, der in einem Dampferzeuger angeordnet ist, oder durch einen Wärmeübertrager, durch den zum Dampferzeuger strömendes Speisewasser geleitet wird, strömen kann. Somit eröffnet dieser Dampfsterilisator einerseits die Möglichkeit, Speisewasser in einem Dampferzeuger zu verdampfen, und andererseits die Möglichkeit, zum Dampferzeuger strömendes Speisewasser vorzuwärmen.

Aus der DE 43 12 474 C1 ist ein Dampfsterilisator bekannt, bei dem ein Wärmeübertrager eingesetzt werden kann. Dabei wird die im Wärmeübertrager rückgewonnene Wärme des aus einer Sterilisationskammer stammenden Dampfs genutzt, um Warmwasser für eine andere Einrichtung zuzubereiten.

Aus der DE 696 22 316 T2 ist eine Anlage zum Sterilisieren mittels feuchter Hitze bekannt, bei der zwei voneinander getrennte und separat mit Dampf beaufschlagbare Wärmeübertrager vorgesehen sind.

Aus der DE 694 23 569 T2 ist eine Sterilisationsanlage für einen Dampfautoklaven bekannt, die ähnlich wie die zuvor diskutierte Sterilisationsanlage aufgebaut ist. Dabei offenbart diese Übersetzung eines europäischen Patents insbesondere verschiedene Sterilisationsverfahren, die mit dieser Anlage durchgeführt werden können.

Die vorstehend diskutierten Dokumente des Stands der Technik beschreiben unterschiedliche Lösungen, wie Wärme aus dem Dampf, der aus einem Druckkessel eines Dampfsterilisators stammt, weiterverwendet werden kann. Dabei weisen diese Lösungen jedoch unterschiedliche Nachteile auf. Teilweise ist eine Abkühlung des Dampfes nur unter Einsatz großer Wassermengen möglich. Teilweise wird die aus dem Dampf stammende Wärme nicht sinnvoll weiterverwendet, sondern führt in erster Linie zu einem Aufheizen einer Umgebung des Dampfsterilisators. Dies ist insbesondere problematisch, wenn der Dampfsterilisator in einem kleinen Raum aufgestellt ist. Denn dann ist regelmäßig ein Klimagerät erforderlich, um die Raumtemperatur wieder abzusenken, wodurch zusätzliche Energie verbraucht wird.

Der vorliegenden Erfindung liegt die Aufgabe zugrunde, einen Dampfsterilisator bereitzustellen, bei dessen Betrieb weniger Energie und Wasser als bei den aus dem Stand der Technik bekannten Dampfsterilisatoren benötigt wird. Darüber hinaus soll ein Verfahren zum Betreiben eines solchen Dampfsterilisators angegeben werden.

Diese Aufgabe wird durch einen Dampfsterilisator mit den Merkmalen des Anspruchs 1 gelöst. Ein solcher Dampfsterilisator weist einen Druckkessel, eine erste Fluidablassleitung, die mit dem Druckkessel verbunden ist, eine zweite Fluidablassleitung, die ebenfalls mit dem Druckkessel verbunden ist, eine Vakuumpumpe und einen Speisewassertank auf. Die Vakuumpumpe, die insbesondere als wasserfreie Vakuumpumpe wie beispielsweise als Membranpumpe ausgestaltet ist, dient dazu, ein Fluid, wie etwa Dampf oder ein Dampf-Wasser-Gemisch, aus dem Druckkessel zu saugen.

Der Speisewassertank dient zur Aufnahme von Speisewasser. Dieses Speisewasser wird benötigt, um Dampf zu erzeugen, der in den Druckkessel eingebracht wird.

Der Dampfsterilisator zeichnet sich erfindungsgemäß dadurch aus, dass die erste Fluidablassleitung durch einen ersten Wärmeübertrager mit ausschließlich indirekter Wärmeübertragung geführt ist. Dabei dient ein durch die erste Fluidablassleitung strömendes Fluid als wärmeabgebendes Medium, während Speisewasser als wärmeaufnehmendes Medium dient. Durch die ausschließlich indirekte Wärmeübertragung wird eine strikte Trennung zwischen dem aus dem Druckkessel abgelassenen Fluid und dem Speisewasser, aus dem nachfolgend noch Dampf erzeugt werden soll, welches dem Druckkessel zugeführt wird, erreicht. Dadurch wird ein Übergang von Fremdstoffen wie etwa Ölen oder Desinfektionsmittelrückständen aus dem aus dem Druckkessel abgelassenen Fluid in das Speisewasser vermieden, wodurch eine hohe Speisewasserreinheit und folglich eine hohe Dampfreinheit gewährleistet werden können.

Durch eine Verwendung des Speisewassers als wärmeaufnehmendes Medium wird eine effiziente Wärmerückgewinnung aus dem Fluid, das aus dem Druckkessel stammt, erreicht. Gleichzeitig wird ein Aufheizen eines Raumes, in dem der Dampfsterilisator aufgestellt ist, vermieden. Durch die Erwärmung des Speisewassers wird eine Reduzierung der nötigen Heizleistung im Dampferzeuger erreicht. Da keine Wärmeabgabe an die Raumluft durch den Wärmeübertrager erfolgt, kann die zulässige Beladungsmenge im Vergleich zu anderen wasserfreien Vakuumsystemen in etwa verdoppelt werden. Somit steigt die Leistungsfähigkeit des erfindungsgemäß beanspruchten Dampfsterilisators signifikant; er kann entsprechend größer als aus dem Stand der Technik bekannte Dampfsterilisatoren gebaut werden.

Durch eine Rückgewinnung der Wärme aus dem Fluid, welches aus dem Druckkessel stammt, wird Energie eingespart, die andernfalls zur Erwärmung des Speisewassers benötigt würde. Schließlich wird durch eine Verwendung des Speisewassers als wärmeaufnehmendes Medium auch der Gehalt an nicht kondensierbaren Gasen im Speisewasser verringert. Denn durch die Temperaturerhöhung des Speisewassers sinkt die Löslichkeit derartiger nicht kondensierbarer Gase im Speisewasser. Dadurch wird eine Verbesserung der Dampfqualität erreicht.

Schließlich wird durch eine Erwärmung des Speisewassers auch eine thermische Desinfektion des Speisewassers erreicht, sodass dieses länger in dem Speisewassertank vorrätig gehalten werden kann, ohne dass eine Kontamination des Speisewassertanks beispielsweise durch die Ausbildung eines Biofilms befürchtet werden müsste. Die durch die Wärmeübertragung aus dem Fluid erreichte Temperatur des Speisewassers liegt beispielsweise in einem Bereich von 50 bis 95 °C, insbesondere 55 bis 90 °C, insbesondere 60 bis 85 °C, insbesondere 65 bis 80 °C, insbesondere 70 bis 75 °C.

Die Verwendung von Speisewasser als wärmeaufnehmendes Medium führt auch zu einer insgesamt höheren Kühlleistung des eingesetzten ersten Wärmeübertragers im Vergleich zu einem Wärmeübertrager, der Luft als Kühlmedium verwendet. Denn die spezifische Wärmekapazität von Luft ist deutlich geringer als die von Wasser. Durch die höhere Kühlleistung ist es möglich, alle Prozesse, bei denen ein Vakuumsystem des Dampfsterilisators durchströmt wird, schneller ablaufen zu lassen. Somit sind kürzere Prozesszeiten möglich, was in einer höheren Nutzerfreundlichkeit resultiert.

Eine kürzere Prozesszeit wird aber insbesondere dadurch erreicht, dass die erste Fluidablassleitung mit einem zweiten Wärmeübertrager verbunden ist, der in Strömungsrichtung eines aus dem Druckkessel durch die erste Fluidablassleitung strömenden Fluid hinter dem ersten Wärmeübertrager angeordnet ist. Dadurch wird erreicht, dass ein unter Druck stehendes Fluid aus dem Druckkessel des Dampfsterilisators sowohl durch den ersten Wärmeübertrager als auch durch den zweiten Wärmeübertrager strömen muss, sodass die in dem Fluid enthaltene Wärme effektiv auf andere Medien übertragen wird. Durch eine derartige serielle Anordnung zweier Wärmeübertrager kann eine besonders leistungsstarke Wärmeübertragung aus dem durch die erste Fluidablassleitung strömenden Fluid erreicht werden.

Der zweite Wärmeübertrager ist zusätzlich mit der zweiten Fluidablassleitung verbunden, sodass es auch möglich ist, ein Fluid aus dem Druckkessel durch die zweite Fluidablassleitung zum zweiten Wärmeübertrager strömen zu lassen. In einer Ausgestaltung ist innerhalb der zweiten Fluidablassleitung kein weiterer Wärmeübertrager vorgesehen. Diese zweite Fluidablassleitung dient jedoch nicht dazu, unter Druck stehendes Fluid aus dem Druckkessel abzulassen, sondern wird insbesondere dann eingesetzt, wenn eine Evakuierung des Druckkessels erfolgen soll, also nur noch verhältnismäßig wenig Fluid aus dem Druckkessel mittels der Vakuumpumpe abgesaugt werden soll. Dann wird durch den zweiten Wärmeübertrager sichergestellt, dass selbst dieses Fluid die in ihm enthaltene Wärme abgibt, bevor es die Vakuumpumpe erreicht. So wird ein unnötiges Aufheizen der Vakuumpumpe vermieden.

In Strömungsrichtung eines aus dem Druckkessel strömenden Fluid sind hinter dem zweiten Wärmeübertrager eine dritte Fluidablassleitung und eine vierte Fluidablassleitung vorgesehen. Das heißt, dass der zweite Wärmeübertrager eingangsseitig strömungstechnisch mit zwei Fluidablassleitungen verbunden ist (nämlich der ersten Fluidablassleitung und der zweiten Fluidablassleitung) und ausgangsseitig ebenfalls mit zwei Fluidablassleitungen (nämlich der dritten Fluidablassleitung und der vierten Fluidablassleitung). Dabei kann eine Strömungsverbindung zwischen dem zweiten Wärmeübertrager und der dritten Fluidablassleitung wahlweise hergestellt oder unterbrochen werden. In gleicher Weise kann eine Strömungsverbindung zwischen dem zweiten Wärmeübertrager und der vierten Fluidablassleitung wahlweise hergestellt oder unterbrochen werden. Das Herstellen bzw. Unterbrechen der Strömungsverbindung kann am einfachsten jeweils mittels eines Ventils erfolgen. Auf diese Weise ist es also möglich, dass ein Fluid aus dem Druckkessel durch die erste Fluidablassleitung und/oder die zweite Fluidablassleitung zum zweiten Wärmeübertrager strömt. Ebenso ist es möglich, dass das Fluid aus dem zweiten Wärmeübertrager durch die dritte Fluidablassleitung und/oder die vierte Fluidablassleitung strömt. Somit können beispielsweise die erste Fluidablassleitung, der zweite Wärmeübertrager und die dritte Fluidablassleitung oder die zweite Fluidablassleitung, der zweite Wärmeübertragung und die vierte Fluidablassleitung in Strömungsverbindung gebracht werden. Es ist möglich, dass die erste Fluidablassleitung und die zweite Fluidablassleitung vor dem zweiten Wärmeübertrager einen gemeinsamen Leitungsabschnitt aufweisen, sodass der zweite Wärmeübertrager nur einen Eingangsanschluss aufweisen muss. Auch durch eine derartige Ausgestaltung ist eine strömungstechnische Verbindung zwischen dem zweiten Wärmeübertrager und der ersten Fluidablassleitung sowie der zweiten Fluidablassleitung sichergestellt.

Ein Ablassen von unter Druck stehendem Fluid aus dem Druckkessel mittels einer wasserfreien Vakuumpumpe ist nicht möglich. Um dennoch für ein Abführen des unter Druck stehenden Fluids aus dem Druckkessel zu sorgen, dient die dritte Fluidablassleitung. Außer einem Ventil, mit dem eine Strömungsverbindung zwischen der dritten Fluidablassleitung und dem zweiten Wärmeübertrager hergestellt oder unterbrochen werden kann, befindet sich vorzugsweise kein weiteres Bauelement in der dritten Fluidablassleitung. Somit kann das aus dem Druckkessel stammende Fluid nach Durchströmen des ersten Wärmeübertragers und des zweiten Wärmeübertragers durch die dritte Fluidablassleitung den Dampfsterilisator verlassen.

Die bereits erwähnte Vakuumpumpe ist innerhalb der vierten Fluidablassleitung angeordnet. Wann immer mittels der Vakuumpumpe ein Fluid aus dem Druckkessel gesaugt wird, strömt dieses also durch den zweiten Wärmeübertrager und die vierte Fluidablassleitung. Das Fluid kann dabei durch die erste Fluidablassleitung und/oder die zweite Fluidablassleitung zum zweiten Wärmeübertrager strömen. Es ist also möglich, das Fluid aus dem Druckkessel mittels der Vakuumpumpe abzusaugen, wobei das Fluid wahlweise durch den ersten Wärmeübertrager strömt oder nicht durch den ersten Wärmeübertrager strömt.

Wie eingangs dargelegt, sind aus dem Stand der Technik zahlreiche Lösungen bekannt, bei denen ein Strömen von Speisewasser erforderlich ist, um eine Wärmeübertragung von abgelassenem Dampf auf zugeführtes Speisewasser zu erreichen. Bei jenen Ausgestaltungen kann eine Wärmeübertragung also nur dann erfolgen, wenn das Speisewasser durch die entsprechende Leitung strömt. Dies ist jedoch nur dann der Fall, wenn Dampf erzeugt werden muss. Folglich kann eine Wärmeübertragung nur während sehr kurzer Abschnitte innerhalb eines Sterilisationszyklus durchgeführt werden. Demgegenüber ist der erfindungsgemäß beanspruchte Dampfsterilisator dafür ausgelegt, eine derartige Wärmeübertragung während des gesamten Sterilisationszyklus zu ermöglichen.

Schließlich ist zum Fördern des Speisewassers beim erfindungsgemäß beanspruchten Dampfsterilisator nur eine einzige Speisewasserpumpe vorgesehen. Durch den Verzicht auf zusätzliche Speisewasserpumpen wird ein besonders einfacher, wartungsarmer und kostengünstiger Aufbau des Dampfsterilisators erreicht.

Durch die Kombination der vorstehend erläuterten Merkmale wird ein Dampfsterilisator bereitgestellt, der weniger Wasser als zahlreiche aus dem Stand der Technik bekannten Dampfsterilisatoren verbraucht. Denn er benötigt keine leistungsstarke Vakuumpumpe wie etwa eine Wasserringpumpe, sondern kann mit einer gewöhnlichen Membranpumpe oder einer anderen wasserfreien Pumpe betrieben werden. Außerdem benötigt er kein besonderes Kühlwasser, sondern setzt nur Speisewasser zum Kühlen von Fluid, welches aus dem Druckkessel abgelassen wird, ein. Dieses Speisewasser wird aber ohnehin für den Betrieb des Dampfsterilisators benötigt.

Der beanspruchte Dampfsterilisator verbraucht darüber hinaus für ein Dampfsterilisationsverfahren weniger Energie als die aus dem Stand der Technik bekannten Dampfsterilisatoren. Denn die einmal zur Dampferzeugung eingesetzte Energie wird durch den ersten Wärmeübertrager effizient auf das Speisewasser übertragen, sodass für eine nachfolgende Dampferzeugung bereits vorgewärmtes Speisewasser eingesetzt wird, wodurch der zusätzliche Energieeintrag reduziert wird. Darüber hinaus erfolgt durch den zweiten Wärmeübertrager eine weitere Abkühlung des aus dem Druckkessel stammenden Fluids, sodass dieses mit einer insgesamt niedrigen Temperatur mit oder ohne Einsatz der Vakuumpumpe aus dem Druckkessel abgelassen werden kann.

Gleichzeitig wird eine hohe Dampfqualität erreicht, da der Anteil an nicht kondensierbaren Gasen im Speisewasser reduziert wird und eine strikte Trennung zwischen gegebenenfalls durch Fremdsubstanzen kontaminiertem Dampf auf der einen Seite und frischem Speisewasser, welches zur Erzeugung neuen Dampfs eingesetzt wird, auf der anderen Seite gewährleistet wird.

Durch eine Verhinderung einer Aufheizung der Atmosphäre eines Raumes, in dem der Dampfsterilisator aufgestellt wird, kann der Dampfsterilisator zudem größer dimensioniert werden, um eine größere Beladungsmenge gleichzeitig sterilisieren zu können. Beispielsweise kann die Sterilisationskammer des Dampfsterilisators in einer Ausgestaltung ein Volumen von mindestens 40 I, insbesondere mindestens 45 I, insbesondere mindestens 50 I, insbesondere mindestens 55 I, insbesondere mindestens 60 I aufweisen. In einer Ausgestaltung weist die Sterilisationskammer des Dampfsterilisators ein Volumen von 18 bis 200 I, insbesondere 20 bis 180 I, insbesondere 25 bis 170 I, insbesondere 30 bis 160 I, insbesondere 40 bis 150 I, insbesondere 50 bis 140 I, insbesondere 60 bis 130 I, insbesondere 70 bis 120 I, insbesondere 80 bis 110 I, insbesondere 90 bis 100 I auf. Dampfsterilisatoren mit derart großen Sterilisationskammern bzw. Druckkesseln waren bislang technisch nicht realisierbar, ohne Wasserringpumpen als Vakuumpumpen einzusetzen. Demgegenüber kann der vorliegend beanspruchte Sterilisator insbesondere mit einer wasserfrei arbeitenden Vakuumpumpe ausgestattet sein, wodurch der Wasserverbrauch des Dampfsterilisators erheblich reduziert wird.

Somit führt die Merkmalskombination des beanspruchten Dampfsterilisators in synergistischer Weise zu einem leistungsstärkeren Dampfsterilisator, der weniger Wasser und Energie als die aus dem Stand der Technik bekannten Dampfsterilisatoren verbraucht.

Durch die effiziente Energierückgewinnung aus dem Fluid, welches aus dem Druckkessel stammt, kann der Dampfsterilisator in einer Variante lediglich mit einer einphasigen Heizung ausgestattet werden, sodass er an eine gewöhnliche 16-A-Steckdose angeschlossen werden kann. Dies reduziert den Installationsaufwand des Dampfsterilisators erheblich. Denn die aus dem Stand der Technik bekannten Dampfsterilisatoren müssen regelmäßig an Drehstrom angeschlossen werden, um die eingesetzten mehrphasigen Heizungen mit ausreichend Energie zur Dampferzeugung zu versorgen.

Falls die Temperatur des Speisewassers im Betrieb des Dampfsterilisators einen vorgebbaren Maximalwert überschritten haben sollte, ist es auch möglich, unter Druck stehendes Fluid aus dem Druckkessel durch die zweite Fluidablassleitung abzulassen. Dann strömt dieses Fluid direkt vom Druckkessel zum zweiten Wärmeübertrager und kann von dort durch die dritte Fluidablassleitung den Dampfsterilisator verlassen. In diesem besonderen Fall wird die in dem Fluid enthaltene Wärmeenergie nur im zweiten Wärmeübertrager teilweise abgeführt. Ein solcher Strömungspfad des unter Druck stehenden Fluids aus dem Druckkessel entspricht aber nicht dem gewöhnlichen Betriebszustand des Dampfsterilisators, da dann die oben beschriebenen vorteilhaften Effekte einer Wärmerückgewinnung nicht erreicht werden können. Durch diesen alternativen Betriebszustand wird jedoch eine Überhitzung des Speisewassers vermieden; es handelt sich mithin um einen bei der beschriebenen Ausgestaltung des Dampfsterilisators möglichen Sicherheits-Betriebszustand.

Eine technisch-konstruktive Auslegung des ersten Wärmeübertragers dahingehend, dass Wärme von dem durch die erste Fluidablassleitung strömenden Fluid auf das Speisewasser unabhängig davon übertragen wird, ob das Speisewasser während des Wärmeübertragungsvorgangs zu einem Dampferzeuger strömt oder nicht, kann beispielsweise dadurch erreicht werden, dass der Wärmeübertrager in dem Speisewassertank angeordnet ist. Daher ist es in einer Variante vorgesehen, dass der erste Wärmeübertrager in dem Speisewassertank angeordnet ist. Dann kann eine effiziente Energieübertragung aus dem Fluid, welches durch die erste Fluidablassleitung strömt, auch dann erreicht werden, wenn gerade kein Dampf aus dem Speisewasser erzeugt werden muss. Denn im Speisewassertank ist stets eine bestimmte Menge Speisewasser vorhanden, das dann auf diese Weise erwärmt wird. Wenn anschließend wieder Dampf benötigt wird und hergestellt werden soll, erfolgt dies unter Verwendung bereits vorgewärmten Speisewassers.

Es gibt allerdings noch weitere technisch-konstruktive Möglichkeiten, um zu erreichen, dass die Wärme von dem durch die erste Fluidablassleitung Fluid unabhängig davon, ob das Speisewasser während des Wärmeübertragungsvorgangs zu einem Dampferzeuger strömt, auf das Speisewasser übertragen werden kann. So ist es in einer Alternative vorgesehen, dass der erste Wärmeübertrager in einem Speisewasserkreislauf angeordnet ist, in dem das Speisewasser mittels der Speisewasserpumpe aus dem Speisewassertank durch den ersten Wärmeübertrager hindurch und wieder zurück zum Speisewassertank gefördert werden kann. Dann erfolgt eine Wärmeübertragung nicht auf das im Speisewassertank vorrätig gehaltene Speisewasser, sondern auf strömendes Speisewasser. Dieses Speisewasser strömt allerdings nicht notwendigerweise zu einem Dampferzeuger, zu dem es nur dann strömt, wenn auch Dampf erzeugt werden soll. Vielmehr strömt das Speisewasser in einem Kreislauf, der unabhängig davon aufrechterhalten werden kann, ob Dampf aus dem Speisewasser erzeugt werden soll oder nicht.

In einer Variante ist in dem Speisewasserkreislauf ein Ventil angeordnet, durch das der Speisewasserkreislauf unterbrochen und eine Strömungsverbindung zwischen dem Speisewassertank und einer zu einem Dampferzeuger führenden Speisewasserleitung hergestellt werden kann. Ein solches Ventil dient also dazu, Speisewasser aus dem Speisewassertank wahlweise im Speisewasserkreislauf strömen zu lassen oder aber dem Dampferzeuger zuzuführen. Ein solches Ventil kann beispielsweise als 3/2-Wegeventil ausgestaltet sein.

Um sicherzustellen, dass der erste Wärmeübertrager stets von Speisewasser durchströmt wird, unabhängig davon, ob das Speisewasser im Speisewasserkreislauf oder aber zum Dampferzeuger geführt wird, ist der erste Wärmeübertrager in einer Variante zwischen dem Ventil und dem Speisewassertank angeordnet.

In einer Variante ist der zweite Wärmeübertrager als Luft-Fluid-Wärmeübertrager ausgestaltet. Dabei dient ein durch die erste oder zweite Fluidablassleitung strömendes Fluid als wärmeabgebendes Medium, während Luft als wärmeaufnehmendes Medium dient. Bei dieser Luft handelt sich typischerweise um die Umgebungsluft eines Raumes, in dem der Dampfsterilisator aufgestellt ist. Dennoch kommt es bei dieser Ausgestaltung nur zu einer geringen Erwärmung der Raumluft, da das durch die jeweilige Fluidablassleitung strömende Fluid bereits durch den ersten Wärmeübertrager geströmt ist und in diesem eine signifikante Abkühlung erfahren hat. Somit dient der zweite Wärmeübertrager lediglich zur weiteren Abkühlung des aus dem Druckkessel stammenden Fluids, um so eine noch geringere Temperatur der Abluft bzw. des entstehenden Kondensats zu erreichen. Der Einsatz eines derartigen Luft-Fluid-Wärmeübertragers bietet sich immer dann an, wenn das durch die erste oder zweite Fluidablassleitung strömende Fluid bereits so weit abgekühlt ist, dass eine weitere effiziente Wärmeübertragung auf das Speisewasser kaum mehr möglich ist.

Um ein Sterilisationsverfahren, das in dem Dampfsterilisator durchgeführt wird, möglichst einfach überwachen zu können, ist es in einer Variante vorgesehen, dass im Speisewassertank und/oder in einem Speisewasserkreislauf und/oder in der ersten Fluidablassleitung und/oder in der zweiten Fluidablassleitung mindestens ein Temperatursensor angeordnet ist. Der Speisewasserkreislauf wird dabei durch eine Rohrleitung definiert, in dem Speisewasser mittels der Speisewasserpumpe aus dem Speisewassertank durch den ersten Wärmeübertrager hindurch und zurück zum Speisewassertank gefördert werden kann. Der Temperatursensor dient dazu, an zumindest einem Ort die Temperatur des aus dem Druckkessel stammenden Fluids bzw. des Speisewassers zu bestimmen, um auf diese Weise beispielsweise Strömungsgeschwindigkeiten des Fluids oder des Speisewassers beeinflussen zu können, um für eine verbesserte Wärmeübertragung vom Fluid auf das Speisewasser zu sorgen.

In einer Variante ist in einer von dem Speisewassertank wegführenden Speisewasserleitung, durch die Speisewasser von der Speisewasserpumpe gepumpt werden kann, eine Vorrichtung zur Erfassung eines Durchflusses von Speisewasser angeordnet. Mit einer solchen Vorrichtung zur Erfassung des Durchflusses lässt sich besonders einfach bestimmen, ob und wieviel Speisewasser durch die Speisewasserleitung strömt. So kann beispielsweise direkt ermittelt werden, wieviel Speisewasser in einem Speisewasserkreislauf strömt oder welcher Volumenfluss dort erreicht wird. Ferner kann so ermittelt werden, wie viel Speisewasser von dem Speisewassertank zum Dampferzeuger strömt.

In einer Variante handelt es sich bei der Vorrichtung zur Erfassung eines Durchflusses um einen Coriolis-Massedurchflussmesser, einen Drall-Durchflussmesser, einen Durchflussmesser, einen Flügelradzähler, einen magnetisch-induktiven Durchflussmesser, eine Messblende, einen mittelbaren Volumenzähler, einen Schraubenradzähler, einen Schwebekörper-Durchflussmesser, eine Staudrucksonde, einen thermischen Massedurchflussmesser, einen Turbinenrad-Durchflussmesser, einen Ultraschall-Durchflussmesser, einen unmittelbaren Volumenzähler, einen Venturi-Durchflussmesser, einen Verdrängungszähler, einen Vortex-Durchflussmesser, einen Vortex-Massedurchflussmesser, einen Wirbeldurchflussmesser oder einen Woltmannzähler.

In einer Variante weist der Dampfsterilisator ein Steuerungsmodul auf, das dazu dient, die Speisewasserpumpe und/oder die Vakuumpumpe in Abhängigkeit von Messwerten zu steuern, die von dem Temperatursensor oder der Vorrichtung zur Erfassung eines Durchflusses gemessen werden. Ist beispielsweise die ermittelte Temperatur des Fluids zu hoch, gleichzeitig aber die Flussrate des Speisewassers im Speisewasserkreislauf gering, kann das Steuerungsmodul dafür sorgen, dass die Speisewasserpumpe ihre Leistung erhöht, um eine größere Menge von Speisewasser in der gleichen Zeit durch den Speisewasserkreislauf zu pumpen, damit ein verbesserter Wärmeübergang zwischen dem Fluid und dem Speisewasser erreicht wird.

Die vorliegende Erfindung betrifft ein Verfahren zum Betrieb eines Dampfsterilisators, welcher die vorstehend erläuterten Merkmale aufweist. Dieses Verfahren zeichnet sich dadurch aus, dass ein Fluid, welches aus einem Druckkessel des Dampfsterilisators bzw. Autoklaven zum Druckablass ohne Einsatz einer Vakuumpumpe ausströmt, durch einen ersten Wärmeübertrager mit ausschließlich indirekter Wärmeübertragung und nachfolgend durch einen zweiten Wärmeübertrager strömt.

Ferner wird das Fluid nach erfolgtem Druckablass zur Evakuierung des Druckkessels mittels einer Vakuumpumpe aus dem Druckkessel gesaugt. Dabei strömt das Fluid dann zumindest durch den zweiten Wärmeübertrager.

Darüber hinaus ist vorgesehen, dass der erste Wärmeübertrager beim Durchströmen des Fluids Wärme von dem Fluid auf das Speisewasser, welches zur Erzeugung von Dampf vorgesehen ist, überträgt. Diese Wärmeübertragung erfolgt dabei unabhängig davon, ob das Speisewasser während des Wärmeübertragungsvorgangs zu einem Dampferzeuger strömt. Schließlich ist vorgesehen, dass das Speisewasser in dem Dampfsterilisator durch eine einzige Speisewasserpumpe gefördert wird.

Dieses Verfahren weist die bereits in Zusammenhang mit dem Dampfsterilisator beschriebenen vorteilhaften Effekte auf, die sich in synergistischer Art und Weise durch ein Zusammenspiel der einzelnen Verfahrensschritte ergeben. Es ist insbesondere mit weniger Wasserverbrauch, einem geringeren Energieverbrauch, einer kürzeren Zykluszeit und einer höheren Sterilisationsleistung als die aus dem Stand der Technik bekannten Sterilisationsverfahren durchführbar. In Bezug auf weitere Effekte wird auf die obigen Erläuterungen verwiesen.

Ein typisches Betriebsverfahren sieht folgendermaßen aus: Nach erfolgter Dampfsterilisation wird ein erstes Ventil zwischen dem Druckkessel und der ersten Fluidablassleitung geöffnet. Dadurch strömt das unter Druck stehende Fluid aus dem Druckkessel durch die erste Fluidablassleitung und den ersten Wärmeübertrager zum zweiten Wärmeübertrager. Zugleich wird ein drittes Ventil in der dritten Fluidablassleitung geöffnet, sodass das Fluid auch den zweiten Wärmeübertrager durchströmt und den Dampfsterilisator durch die dritte Fluidablassleitung verlässt.

Wenn der Druck im Druckkessel ein vorgebbares Niveau unterschritten hat (beispielsweise 1500 mbar, insbesondere 1400 mbar, insbesondere 1300 mbar, insbesondere 1200 mbar, insbesondere 1100 mbar, insbesondere 1000 mbar), wird zusätzlich ein zweites Ventil zwischen dem Druckkessel und der zweiten Fluidablassleitung geöffnet. Dann strömt das in dem Druckkessel noch enthaltene Fluid sowohl durch die erste Fluidablassleitung (und durch den ersten Wärmeübertrager) als auch durch die zweite Fluidablassleitung zum zweiten Wärmeübertrager. Zugleich wird das dritte Ventil geschlossen und ein viertes Ventil in der vierten Fluidablassleitung geöffnet, sodass die ebenfalls in der vierten Fluidablassleitung angeordnete Vakuumpumpe für ein Absaugen des Fluid aus dem Druckkessel sorgt. Denn das Fluid, dessen Druck unter dem vorgebbaren Niveau liegt, kann nicht mehr als ein unter Druck stehendes Fluid angesehen werden, das selbsttätig aus dem Druckkessel ausströmt. Vielmehr bedarf es zu seiner Entfernung aus dem Druckkessel der Vakuumpumpe. Nachfolgend wird das erste Ventil geschlossen, sodass das noch im Druckkessel befindliche Fluid direkt vom Druckkessel zum zweiten Wärmeübertrager und von dort zur Vakuumpumpe gesaugt wird. Ein Durchströmen des ersten Wärmeübertragers erfolgt dann nicht mehr. Denn zum jetzigen Zeitpunkt noch wenig Fluid aus dem Druckkessel entfernt, sodass keine effiziente Wärmeübertragung auf das Speisewasser mehr möglich wäre.

Das aus dem Druckkessel ausströmende und unter Druck stehende Fluid muss nach Durchströmen des ersten Wärmeübertragers durch den zweiten Wärmeübertrager strömen. Das heißt, dass das Fluid durch zwei seriell hintereinander geschaltete Wärmeübertrager geführt wird, was in einer besonders effizienten Wärmeübertragung aus dem Fluid resultiert.

In einer Variante ist der zweite Wärmeübertrager ein Luft-Fluid-Wärmeübertrager, wobei der zweite Wärmeübertrager Wärme von dem durch ihn strömenden Fluid auf Luft überträgt. Bei dieser Luft kann es sich um Umgebungsluft handeln.

In einer Variante werden die Speisewasserpumpe und/oder die Vakuumpumpe des Dampfsterilisators in Abhängigkeit von Messwerten gesteuert, die von mindestens einem Temperatursensor oder mindestens einer Vorrichtung zur Erfassung eines Durchflusses, insbesondere eines Durchflusses von Speisewasser, gemessen werden. Dann lassen sich die Speisewasserpumpe und/oder die Vakuumpumpe besonders einfach an konkrete verfahrenstechnische Bedingungen des Sterilisationsprozesses anpassen.

In einer Variante misst der Temperatursensor die Temperatur des Speisewassers in einem Speisewassertank, die Temperatur des Speisewassers in einem Speisewasserkreislauf und/oder die Temperatur des aus dem Druckkessel ausströmenden Fluids. In dem Speisewasserkreislauf wird das Speisewasser dabei mittels der Speisewasserpumpe aus dem Speisewassertank durch den ersten Wärmeübertrager hindurch und wieder zurück zum Speisewassertank gefördert. Die Temperatur des Fluids wird in einer Variante nach Durchströmen des ersten Wärmeübertragers gemessen. Außerdem ist es möglich, die Temperatur des Fluids nach Durchströmen des zweiten Wärmeübertragers zu messen.

In einer Variante misst die Vorrichtung zur Erfassung eines Durchflusses einen Volumenstrom des Speisewassers, welches von der Speisewasserpumpe aus dem Speisewassertank gepumpt wird. Dann lässt sich besonders einfach ermitteln, welche Wärmemenge von diesem Speisewasser aufgenommen werden kann, wenn es im ersten Wärmeübertrager mit Wärme aus dem durch die erste Fluidablassleitung strömenden Fluid beaufschlagt wird.

Sämtliche der vorstehend erläuterten Varianten und Ausgestaltungen des beschriebenen Dampfsterilisators können in beliebiger Weise miteinander kombiniert und in jeder Kombination auf das beschriebene Verfahren übertragen werden, und umgekehrt.

Weitere Details von Aspekten der vorliegenden Erfindung sollen anhand von Ausführungsbeispielen und Figuren näher erläutert werden. Es zeigen:
- Figur 1: eine schematische Darstellung eines ersten Ausführungsbeispiels eines Dampfsterilisators und
- Figur 2: eine schematische Darstellung eines zweiten Ausführungsbeispiel eines Dampfsterilisators.

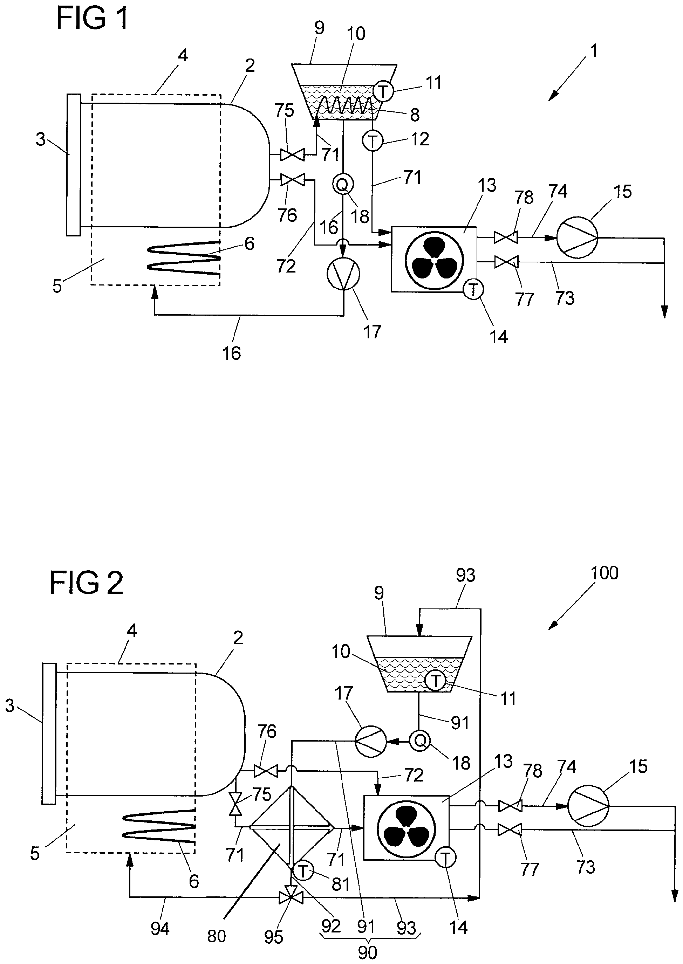

Die Figur 1 zeigt eine schematische Ansicht eines Leitungsbildes eines Dampfsterilisators 1 mit einem Druckkessel 2. Dieser Druckkessel 2 weist eine Tür 3 auf, durch die zu sterilisierende Gegenstände in den Druckkessel 2 hineingelegt und aus dem Druckkessel 2 wieder entnommen werden können. Um den Druckkessel 2 herum ist mantelartig ein Dampferzeuger 4 angeordnet, der in seinem unteren Bereich ein Wasserreservoir 5 und eine in dem Wasserreservoir 5 angeordnete Heizung 6 aufweist. Durch die Heizung 6 kann das im Wasserreservoir 5 vorhandene Wasser verdampft werden, woraufhin es anschließend aus dem Dampferzeuger 4 in den Druckkessel 2 geleitet wird.

Wenn ein Druck, der in dem Druckkessel 2 aufgebaut wurde, abgelassen werden soll, kann der in dem Druckkessel 2 enthaltene Dampf durch eine erste Dampfablassleitung 71 abgelassen werden. Diese erste Dampfablassleitung 71 dient als erste Fluidablassleitung und kann auch als Druckablassleitung bezeichnet werden. Damit Dampf durch die erste Dampfablassleitung 71 strömen kann, muss ein erstes Ventil 75 geöffnet sein. Der Dampf, der durch die erste Dampfablassleitung 71 strömt, gelangt zu einem Fluid-Wasser-Wärmeübertrager 8, der als erster Wärmeübertrager dient. Der Fluid-Wasser-Wärmeübertrager 8 ist dabei in einem Speisewassertank 9 angeordnet, und zwar derart, dass er Wärme aus dem Dampf, der durch die erste Dampfablassleitung 71 strömt, auf Speisewasser 10, welches im Speisewassertank 9 vorrätig gehalten wird, überträgt. Da der Fluid-Wasser-Wärmeübertrager 8 fluiddicht gegenüber dem Speisewasser 10 abgeschlossen ist, kommt es lediglich zu einer Energieübertragung von dem Dampf auf das Speisewasser 10, nicht jedoch zu einer Stoffübertragung.

Die Temperatur des Speisewassers 10 wird durch einen ersten Temperatursensor 11, der im Speisewassertank 9 angeordnet ist, überwacht.

Ein zweiter Temperatursensor 12 befindet sich in der ersten Dampfablassleitung 71 hinter dem Fluid-Wasser-Wärmeübertrager 8. Mittels dieses zweiten Temperatursensors 12 kann die Temperatur des im Fluid-Wasser-Wärmeübertrager 8 bereits abgekühlten Dampfs ermittelt werden.

Der durch die erste Dampfablassleitung 71 strömende Dampf bzw. das nunmehr durch diese Leitung strömende Dampf-Wasser-Gemisch gelangt anschließend zu einem Luftkühler 13, der als zweiter Wärmeübertrager dient. Er ist dabei als Luft-Fluid-Wärmeübertrager ausgestaltet.

In diesem Luftkühler 13 wird das Dampf-Wasser-Gemisch weiter abgekühlt, wobei seine Temperatur durch einen dritten Temperatursensor 14 überwacht wird. Hinter dem Luftkühler 13 strömt das abgekühlte Dampf-Wassergemisch bzw. Kondensat durch eine dritte Dampfablassleitung 73 aus dem Dampfsterilisator 1, sofern ein drittes Ventil 77 geöffnet ist.

Wenn der Druck des Dampfs im Druckkessel 2 unter einen bestimmten Schwellenwert sinkt, wird eine Vakuumpumpe 15 aktiviert, um den Dampf aus dem Druckkessel 2 zu fördern. Diese Vakuumpumpe 15 ist in einer vierten Dampfablassleitung 74 angeordnet, wobei zwischen der vierten Dampfablassleitung 74 und dem Luftkühler 13 ein viertes Ventil 78 angeordnet ist, mit dem eine Strömungsverbindung zwischen dem Luftkühler 13 und der vierten Dampfablassleitung 74 hergestellt oder unterbrochen werden kann.

Wenn die Vakuumpumpe 15 aktiviert wird, wird zudem ein zweites Ventil 76 geöffnet, mit dem eine Strömungsverbindung zwischen dem Druckkessel 2 und einer zweiten Dampfablassleitung 72 hergestellt wird. Der Dampf kann dann aus dem Druckkessel 2 sowohl über die erste Dampfablassleitung 71 (und damit durch den Fluid-Wasser-Wärmeübertrager 8) als auch direkt durch die zweite Dampfablassleitung 72 zum Luftkühler 13 strömen. Wenn der Druck des Dampfs im Druckkessel 2 weiter absinkt, wird das erste Ventil 75 geschlossen, sodass der Dampf dann aus dem Druckkessel 2 ausschließlich durch die zweite Dampfablassleitung 72, den Luftkühler 13 und die vierte Dampfablassleitung 74 abgesaugt wird, um im Druckkessel 2 einen Unterdruck zu erzeugen.

Der durch die unterschiedlichen Dampfablassleitungen strömende Dampf bzw. das durch diese Leitungen strömende Dampf-Wasser-Gemisch wird durch den Fluid-Wasser-Wärmeübertrager 8 und den Luftkühler 13 letztlich gänzlich kondensiert und in dieser gänzlich kondensierten Form einer Abwasserleitung zugeführt.

Das mithilfe des Fluid-Wasser-Wärmeübertragers 8 im Speisewassertank 9 erwärmte Speisewasser 10 kann immer dann, wenn der Wasserstand im Wasserreservoir 5 zu niedrig geworden ist, durch eine Speisewasserleitung 16 mittels einer Speisewasserpumpe 17 aus dem Speisewassertank 9 in das Wasserreservoir 5 gepumpt werden. Die Speisewasserpumpe 17 ist die einzige Speisewasserpumpe des Dampfsterilisators 1. Ein Durchflussmesser 18 in der Speisewasserleitung 16 dient dabei als Vorrichtung zur Erfassung eines Durchflusses des Speisewassers. Mittels des Durchflussmessers 18 ist es möglich, einen Volumenstrom des Speisewassers, der von der Speisewasserpumpe 17 gepumpt wird, zu erfassen. Auf diese Weise lässt sich leicht die Menge des Speisewassers bestimmen, das aus dem Speisewassertank 9 in das Wasserreservoir 5 des Druckerzeugers 4 gepumpt wird.

Da das Speisewasser 10 bereits im Speisewassertank 9 vorgewärmt wurde, benötigt die Heizung 6 weniger Heizenergie, um aus dem Speisewasser 10 im Wasserreservoir 5 Dampf zu erzeugen. Darüber hinaus sorgt die spezifische Anordnung von Wasserreservoir 5, Speisewassertank 9 und Speisewasserpumpe 17 dafür, dass eine Wärmeübertragung von dem Dampf, der durch die erste Dampfablassleitung 71 strömt, auf das Speisewasser 10 stets möglich ist, unabhängig davon, ob das Speisewasser 10 gerade vom Speisewassertank 9 zum Wasserreservoir 5 gepumpt wird oder nicht. Die Temperatur des Speisewassers 10 im Speisewassertank 9 kann dabei beispielsweise auf bis zu 90 °C ansteigen. Dann wird bereits eine thermische Desinfektion des Speisewassers 10 im Speisewassertank 9 erreicht, wodurch eine Biofilmbildung im Speisewassertank 9 vermieden werden kann. Die Restwärme, die im Dampf bzw. im Dampf-Wasser-Gemisch nach dem Fluid-Wasser-Wärmeübertrager 8 noch enthalten ist, ist verhältnismäßig gering, sodass beim Einsatz des Luftkühlers 13 nur noch eine geringe Erwärmung der Umgebungsluft des Dampfsterilisators 1 erfolgt.

Die Figur 2 zeigt eine schematische Darstellung der Leitungen eines weiteren Dampfsterilisators 100, der sich nur gering von dem Dampfsterilisator 1 der Figur 1 unterscheidet. Daher werden die gleichen Elemente auch mit den gleichen Bezugszeichen versehen. Es wird insoweit auf die obigen Erläuterungen verwiesen. Nachfolgend werden lediglich die Unterschiede zwischen den Dampfsterilisator 100 der Figur 2 und dem Dampfsterilisator 1 der Figur 1 erläutert.

Bei dem in der Figur 2 schematisch dargestellten Dampfsterilisator 100 ist ebenfalls ein Fluid-Wasser-Wärmeübertrager 80 vorgesehen, der jedoch nicht innerhalb des Speisewassertanks 9 angeordnet ist. Vielmehr befindet sich dieser Fluid-Wasser-Wärmeübertrager 80 in einem Speisewasserkreislauf 90, der aus verschiedenen Speisewasserleitungsabschnitten besteht. Konkret ist ein erster Speisewasserleitungsabschnitt 91 vorgesehen, der den Speisewassertank 9 mit dem Fluid-Wasser-Wärmeübertrager 80 verbindet. Innerhalb des ersten Speisewasserleitungsabschnitts 91 sind zudem der Durchflussmesser 18 und die Speisewasserpumpe 17 angeordnet.

Ferner ist ein zweiter Speisewasserleitungsabschnitt 92 vorgesehen, der den Fluid-Wasser-Wärmeübertrager 80 mit einem 3/2-Wegeventil 95 verbindet. Vom 3/2-Wegeventil 95 führt ein dritter Speisewasserleitungsabschnitt 93 wieder zurück zum Speisewassertank 9. Somit ist es möglich, dass das Speisewasser 10 aus dem Speisewassertank 9 durch den ersten Speisewasserleitungsabschnitt 91, den Fluid-Wasser-Wärmeübertrager 80, den zweiten Speisewasserleitungsabschnitt 92, das 3/2-Wegeventil 95 und den dritten Speisewasserleitungsabschnitt 93 zurück zum Speisewassertank 9 strömt. Ein derartiges Strömen wird dabei durch die Speisewasserpumpe 17 bewerkstelligt.

Wenn das Speisewasser 10 durch den Speisewasserkreislauf 90 gepumpt wird, wird es im Fluid-Wasser-Wärmeübertrager 80 erwärmt, wenn durch diesen Wärmeübertrager Dampf bzw. ein Dampf-Wasser-Gemisch aus dem Druckkessel 2 des Dampfsterilisators 100 strömt. Dabei erfolgt diese Erwärmung des Speisewassers 10 unabhängig davon, ob dem Wasserreservoir 5 des Dampferzeugers 4 Wasser zugeführt werden muss, um Dampf zu erzeugen. Vielmehr kann ein unabhängig vom Dampferzeuger 4 funktionierender Speisewasserstrom im Speisewasserkreislauf 90 aufgebaut werden.

Soll dem Wasserreservoir 5 des Dampferzeugers 4 hingegen Speisewasser zugeführt werden, wird das 3/2-Wegeventil 95 derart umgestaltet, dass das Speisewasser 10 nun vom Speiswassertank 9 zum Wasserreservoir 5 strömt. Auch dabei strömt es durch den Fluid-Wasser-Wärmeübertrager 80, sodass es weiterhin von Dampf bzw. einem Dampf-Wasser-Gemisch, welches durch die erste Dampfablassleitung 71 strömt, erwärmt wird.

An einer Ausgangsseite des Fluid-Wasser-Wärmeübertragers 80 ist zudem ein vierter Temperatursensor 81 vorgesehen, mit dem die Temperatur des Speisewassers, welches den Fluid-Wasser-Wärmeübertrager 80 durchströmt hat, ermittelt werden kann. Dieser Prozessparameter kann dann von einem Steuerungsmodul des Dampfsterilisators 100 dazu verwendet werden, den Durchfluss des durch den Speisewasserkreislauf 90 oder durch einen vierten Speisewasserleitungsabschnitt 94 zum Wasserreservoir 5 strömenden Speisewassers 10 zu erhöhen oder zu erniedrigen. Dieser vierte Speisewasserleitungsabschnitt 94 stellt dabei keinen Teil des Speisewasserkreislaufs 90 da, sondern ist ein unabhängig vom Speisewasserkreislauf 90 mit dem 3/2-Wegeventil 95 verbundener Speisewasserleitungsabschnitt.

Unabhängig davon, ob der Fluid-Wasser-Wärmeübertrager im Ausführungsbeispiel der Figur 1 innerhalb des Speisewassertanks 9 oder - wie im Ausführungsbeispiel der Figur 2 - im Speisewasserkreislauf 90 angeordnet ist, lassen sich durch die beschriebenen Ausführungsbeispiele eines Dampfsterilisators zahlreiche Vorteile erzielen, auf die im Zusammenhang mit der Erläuterung der Erfindung bereits weiter oben eingegangen wurde. Insbesondere arbeiten die Dampfsterilisatoren mit einem geringeren Wasserverbrauch, einem geringeren Energieeintrag, einer effizienteren Speisewasser-Vorwärmung und kürzeren Prozesszeiten als aus dem Stand der Technik bekannte Dampfsterilisatoren.

## Patentansprüche

1. Dampfsterilisator mit einem Druckkessel (2), einer mit dem Druckkessel (2) verbundenen ersten Fluidablassleitung (71), einer mit dem Druckkessel (2) verbundenen zweiten Fluidablassleitung (72), einer Vakuumpumpe (15), die mit mindestens einer der ersten Fluidablassleitung (71) und der zweiten Fluidablassleitung (72) in Strömungsverbindung gebracht werden kann und mittels der ein Fluid aus dem Druckkessel (2) abgesaugt werden kann, und mit einem Speisewassertank (9), der zur Aufnahme von Speisewasser (10) dient, welches zur Erzeugung von Dampf vorgesehen ist, der dem Druckkessel (2) zugeführt werden kann,
**dadurch gekennzeichnet,**
**dass** die erste Fluidablassleitung (71) durch einen ersten Wärmeübertrager (8, 80) mit ausschließlich indirekter Wärmeübertragung geführt ist, wobei ein durch die erste Fluidablassleitung (71) strömendes Fluid als wärmeabgebendes Medium dient und Speisewasser (10) als wärmeaufnehmendes Medium dient,
**dass** die erste Fluidablassleitung (71) und die zweite Fluidablassleitung (72) in Strömungsverbindung mit einem zweiten Wärmeübertrager (13) stehen, der in Strömungsrichtung eines aus dem Druckkessel (2) durch die erste Fluidablassleitung (71) strömenden Fluids hinter dem ersten Wärmeübertrager (8, 80) angeordnet ist,
**dass** der Dampfsterilisator (1, 100) in Strömungsrichtung eines aus dem Druckkessel strömenden Fluids hinter dem zweiten Wärmeübertrager (13) eine dritte Fluidablassleitung (73) und eine vierte Fluidablassleitung (74) aufweist, wobei wahlweise eine Strömungsverbindung zwischen der dritten Fluidablassleitung (73) und dem zweiten Wärmeübertrager (13) oder eine Strömungsverbindung zwischen der vierten Fluidablassleitung (74) und dem zweiten Wärmeübertrager (13) hergestellt oder unterbrochen werden kann,
**dass** die Vakuumpumpe (15) in der vierten Fluidablassleitung (74) angeordnet ist, und
**dass** zum Fördern des Speisewassers (10) in dem Dampfsterilisator (1, 100) eine einzige Speisewasserpumpe (17) vorgesehen ist.

2. Dampfsterilisator nach Anspruch 1, **dadurch gekennzeichnet, dass** der erste Wärmeübertrager (8) in dem Speisewassertank (9) angeordnet ist.

3. Dampfsterilisator nach Anspruch 1, **dadurch gekennzeichnet, dass** der erste Wärmeübertrager (80) in einem Speisewasserkreislauf (90) angeordnet ist, in dem Speisewasser (10) mittels der Speisewasserpumpe (17) aus dem Speisewassertank (9) durch den ersten Wärmeübertrager (80) hindurch und zurück zum Speisewassertank (9) gefördert werden kann.

4. Dampfsterilisator nach Anspruch 3, **dadurch gekennzeichnet, dass** in dem Speisewasserkreislauf (90) ein Ventil (95) angeordnet ist, durch das der Speisewasserkreislauf (90) unterbrochen und eine Strömungsverbindung zwischen dem Speisewassertank (9) und einer zu einem Dampferzeuger (4) führenden Speisewasserleitung (94) hergestellt werden kann.

5. Dampfsterilisator nach einem der vorhergehenden Ansprüche, **dadurch gekennzeichnet, dass** der zweite Wärmeübertrager (13) ein Luft-Fluid-Wärmeübertrager ist, wobei ein durch die erste Fluidablassleitung (71) und/oder die zweite Fluidablassleitung (72) zum zweiten Wärmeübertrager (13) geleitetes Fluid als wärmeabgebendes Medium dient und Luft als wärmeaufnehmendes Medium dient.

6. Dampfsterilisator nach einem der vorhergehenden Ansprüche, **dadurch gekennzeichnet, dass** im Speisewassertank (9), in einer Speisewasserleitung (16, 91-94), in einem Speisewasserkreislauf (90), in dem Speisewasser (10) mittels der Speisewasserpumpe (17) aus dem Speisewassertank (9) durch den ersten Wärmeübertrager (80) hindurch und zurück zum Speisewassertank (9) gefördert werden kann, in der ersten Fluidablassleitung (71) und/oder in der zweiten Fluidablassleitung (72) ein Temperatursensor (11, 12, 14, 81) angeordnet ist.

7. Dampfsterilisator nach einem der vorhergehenden Ansprüche, **dadurch gekennzeichnet, dass** in einer von dem Speisewassertank (9) wegführenden Speisewasserleitung (16, 91, 92, 94), durch die Speisewasser (10) von der Speisewasserpumpe (17) gepumpt werden kann, eine Vorrichtung (18) zur Erfassung eines Durchflusses angeordnet ist.

8. Verfahren zum Betrieb des Dampfsterilisators (1, 100) nach einem der vorhergehenden Ansprüche,
**dadurch gekennzeichnet,**
**dass** ein Fluid, das zum Druckablass ohne Einsatz einer Vakuumpumpe (15) aus einem Druckkessel (2) des Dampfsterilisators (1, 100) ausströmt, durch einen ersten Wärmeübertrager (8, 80) mit ausschließlich indirekter Wärmeübertragung und nachfolgend durch einen zweiten Wärmeübertrager (13) strömt,
**dass** das Fluid nach erfolgtem Druckablass zur Evakuierung des Druckkessels (2) mittels einer Vakuumpumpe (15) aus dem Druckkessel gesaugt wird, wobei das derart abgesaugte Fluid zumindest durch den zweiten Wärmeübertrager (13) strömt,
**dass** der erste Wärmeübertrager (8, 80) beim Durchströmen des Fluids Wärme von dem Fluid auf Speisewasser (10), welches zur Erzeugung von Dampf vorgesehen ist, überträgt, unabhängig davon, ob das Speisewasser (10) während des Wärmeübertragungsvorgangs zu einem Dampferzeuger (4) strömt, und
**dass** das Speisewasser (10) in dem Dampfsterilisator (1, 100) durch eine einzige Speisewasserpumpe (17) gefördert wird.

9. Verfahren nach Anspruch 8, **dadurch gekennzeichnet, dass** der zweite Wärmeübertrager (13) ein Luft-Fluid-Wärmeübertrager ist, wobei der zweite Wärmeübertrager (13) beim Durchströmen des Fluids Wärme von dem Fluid auf Luft überträgt.

## Claims

1. Steam sterilizer with a pressure vessel (2), a first fluid discharge line (71) which is connected to the pressure vessel (2), a second fluid discharge line (72) which is connected to the pressure vessel (2), a vacuum pump (15) which can be brought into a flow connection with at least one of the first fluid discharge line (71) and the second fluid discharge line (72), and by means of which vacuum pump (15) a fluid can be extracted from the pressure vessel (2), and with a feed water tank (9) which serves to receive feed water (10) which is provided for the generation of steam which can be fed to the pressure vessel (2),
**characterized**
**in that** the first fluid discharge line (71) is routed through a first heat exchanger (8, 80) with exclusively indirect heat transfer, a fluid which flows through the first fluid discharge line (71) serving as heat-emitting medium, and feed water (10) serving as heat-absorbing medium,
**in that** the first fluid discharge line (71) and the second fluid discharge line (72) are in a flow connection with a second heat exchanger (13) which is arranged downstream of the first heat exchanger (8, 80) in the flow direction of a fluid which flows out of the pressure vessel (2) through the first fluid discharge line (71),
**in that** the steam sterilizer (1, 100) has a third fluid discharge line (73) and a fourth fluid discharge line (74) downstream of the second heat exchanger (13) in the flow direction of a fluid which flows out of the pressure vessel, it being possible for a flow connection between the third fluid discharge line (73) and the second heat exchanger (13) or a flow connection between the fourth fluid discharge line (74) and the second heat exchanger (13) to be selectively established or interrupted,
**in that** the vacuum pump (15) is arranged in the fourth fluid discharge line (74), and
**in that** a single feed water pump (17) is provided for conveying the feed water (10) in the steam sterilizer (1, 100).

2. Steam sterilizer according to Claim 1, **characterized in that** the first heat exchanger (8) is arranged in the feed water tank (9).

3. Steam sterilizer according to Claim 1, **characterized in that** the first heat exchanger (80) is arranged in a feed water circuit (90), in which feed water (10) can be conveyed by means of the feed water pump (17) out of the feed water tank (9) through the first heat exchanger (80) and back to the feed water tank (9).

4. Steam sterilizer according to Claim 3, **characterized in that** a valve (95) is arranged in the feed water circuit (90), by way of which valve (95) the feed water circuit (90) can be interrupted and a flow connection between the feed water tank (9) and a feed water line (94) which leads to a steam generator (4) can be established.

5. Steam sterilizer according to one of the preceding claims, **characterized in that** the second heat exchanger (13) is an air/fluid heat exchanger, a fluid which is conducted through the first fluid discharge line (71) and/or the second fluid discharge line (72) to the second heat exchanger (13) serving as heat-emitting medium, and air serving as heat-absorbing medium.

6. Steam sterilizer according to one of the preceding claims, **characterized in that** a temperature sensor (11, 12, 14, 81) is arranged in the feed water tank (9), in a feed water line (16, 91-94), in a feed water circuit (90), in which feed water (10) can be conveyed by means of the feed water pump (17) out of the feed water tank (9) through the first heat exchanger (80) and back to the feed water tank (9), in the first fluid discharge line (71) and/or in the second fluid discharge line (72).

7. Steam sterilizer according to one of the preceding claims, **characterized in that** an apparatus (18) for detecting a throughflow is arranged in a feed water line (16, 91, 92, 94) which leads away from the feed water tank (9) and through which feed water (10) can be pumped by the feed water pump (17).

8. Method for operating the steam sterilizer (1, 100) according to one of the preceding claims,
**characterized**
**in that** a fluid which, for the pressure discharge, without the use of a vacuum pump (15), flows out of a pressure vessel (2) of the steam sterilizer (1, 100) flows through a first heat exchanger (8, 80) with exclusively indirect heat transfer and subsequently through a second heat exchanger (13),
**in that**, after the pressure discharge has taken place, the fluid is sucked from the pressure vessel by means of a vacuum pump (15) in order to evacuate the pressure vessel (2), the fluid which is extracted in this way flowing at least through the second heat exchanger (13), in that, during the throughflow of the fluid, the first heat exchanger (8, 80) transfers heat from the fluid to feed water (10) which is provided for the generation of steam, regardless of whether the feed water (10) flows to a steam generator (4) during the heat transfer process, and
**in that** the feed water (10) in the steam sterilizer (1, 100) is conveyed by way of a single feed water pump (17).

9. Method according to Claim 8, **characterized in that** the second heat exchanger (13) is an air/fluid heat exchanger, the second heat exchanger (13) transferring heat from the fluid to air during the throughflow of the fluid.

## Revendications

1. Stérilisateur à vapeur comprenant un récipient sous pression (2), une première conduite de vidange de fluide (71) reliée au récipient sous pression (2), une deuxième conduite de vidange de fluide (72) reliée au récipient sous pression (2), une pompe à vide (15) qui peut être reliée fluidiquement à l'une au moins parmi la première conduite de vidange de fluide (71) et la deuxième conduite de vidange de fluide (72) et qui permet d'aspirer un fluide dans le récipient sous pression (2), et un réservoir d'eau d'alimentation (9) qui sert à recevoir de l'eau d'alimentation (10) qui est destinée pour la génération de vapeur qui peut être amenée au récipient sous pression (2),
**caractérisé en ce que**
la première conduite de vidange de fluide (71) est guidée à travers un premier échangeur de chaleur (8, 80) à transfert de chaleur exclusivement indirect, un fluide qui s'écoule à travers la première conduite de vidange de fluide (71) servant de milieu exothermique et l'eau d'alimentation (10) servant de milieu endothermique,
la première conduite de vidange de fluide (71) et la deuxième conduite de vidange de fluide (72) sont reliées fluidiquement à un deuxième échangeur de chaleur (13) qui est disposé en aval du premier échangeur de chaleur (8, 80) dans le sens d'écoulement d'un fluide qui sort du récipient sous pression (2) et qui s'écoule à travers la première conduite de vidange de fluide (71),
le stérilisateur à vapeur (1, 100) comporte une troisième conduite de vidange de fluide (73) et une quatrième conduite de vidange de fluide (74) en aval du deuxième échangeur de chaleur (13) dans le sens d'écoulement d'un fluide qui sort du récipient sous pression, une liaison fluidique entre la troisième conduite de vidange de fluide (73) et le deuxième échangeur de chaleur (13) ou une liaison fluidique entre la quatrième conduite de vidange de fluide (74) et le deuxième échangeur de chaleur (13) pouvant au choix être établie ou interrompue,
la pompe à vide (15) est disposée dans la quatrième conduite de vidange de fluide (74), et
une seule pompe à eau d'alimentation (17) est prévue pour transporter l'eau d'alimentation (10) dans le stérilisateur à vapeur (1, 100).

2. Stérilisateur à vapeur selon la revendication 1, **caractérisé en ce que** le premier échangeur de chaleur (8) est disposé dans le réservoir d'eau d'alimentation (9) .

3. Stérilisateur à vapeur selon la revendication 1, **caractérisé en ce que** le premier échangeur de chaleur (80) est disposé dans un circuit d'eau d'alimentation (90) dans lequel l'eau d'alimentation (10) peut être transportée du réservoir d'eau d'alimentation (9) à travers le premier échangeur de chaleur (80), et ramenée au réservoir d'eau alimentaire (9), par la pompe à eau (17).

4. Stérilisateur à vapeur selon la revendication 3, **caractérisé en ce qu**'une vanne (95) est disposée dans le circuit d'eau d'alimentation (90), laquelle vanne permet d'interrompre le circuit d'eau d'alimentation (90) et d'établir une liaison fluidique entre le réservoir d'eau d'alimentation (9) et une conduite d'eau d'alimentation (94) menant à un générateur de vapeur (4).

5. Stérilisateur à vapeur selon l'une quelconque des revendications précédentes, **caractérisé en ce que** le deuxième échangeur de chaleur (13) est un échangeur de chaleur air-fluide, un fluide qui est conduit à travers la première conduite de vidange de fluide (71) et/ou la deuxième conduite de vidange de fluide (72) vers le deuxième échangeur de chaleur (13) servant de milieu exothermique et l'air servant de milieu endothermique.

6. Stérilisateur à vapeur selon l'une quelconque des revendications précédentes, **caractérisé en ce qu**'un capteur de température (11, 12, 14, 81) est disposé dans le réservoir d'eau d'alimentation (9), dans une conduite d'eau d'alimentation (16, 91-94), dans un circuit d'eau d'alimentation (90) dans lequel l'eau d'alimentation (10) peut être transportée du réservoir d'eau d'alimentation (9) à travers le premier échangeur de chaleur (80), et ramenée au réservoir d'eau d'alimentation (9), au moyen de la pompe à eau d'alimentation (17), dans la première conduite de vidange de fluide (71) et/ou dans la deuxième conduite de vidange de fluide (72).

7. Stérilisateur à vapeur selon l'une quelconque des revendications précédentes, **caractérisé en ce qu**'un dispositif (18) de détection d'un débit est disposé dans une conduite d'eau d'alimentation (16, 91, 92, 94) qui part du réservoir d'eau d'alimentation (9) et à travers laquelle l'eau d'alimentation (10) peut être pompée par la pompe à eau d'alimentation (17).

8. Procédé de fonctionnement du stérilisateur à vapeur (1, 100) selon l'une quelconque des revendications précédentes,
**caractérisé en ce que**
un fluide, qui s'écoule d'un récipient sous pression (2) du stérilisateur à vapeur (1, 100) pour le purge de pression sans pompe à vide (15), s'écoule à travers un premier échangeur de chaleur (8, 80) à transfert de chaleur exclusivement indirect puis à travers un deuxième échangeur de chaleur (13),
le fluide est aspiré du récipient sous pression, après que le purge de pression a été effectué, afin de faire le vide dans le récipient sous pression (2) au moyen d'une pompe à vide (15), le fluide ainsi aspiré s'écoulant au moins à travers le deuxième échangeur de chaleur (13),
le premier échangeur de chaleur (8, 80) transfère, lors du passage du fluide, la chaleur du fluide à l'eau d'alimentation (10) qui est prévue pour la génération de vapeur, indépendamment du fait que l'eau d'alimentation (10) s'écoule vers un générateur de vapeur (4) pendant le processus de transfert de chaleur, et
l'eau d'alimentation (10) dans le stérilisateur à vapeur (1, 100) est transportée par une seule pompe d'eau d'alimentation (17).

9. Procédé selon la revendication 8, **caractérisé en ce que** le deuxième échangeur de chaleur (13) est un échangeur de chaleur air-fluide, le deuxième échangeur de chaleur (13) transférant la chaleur du fluide à l'air lors de l'écoulement du fluide.
